# EUROPEAN PATENT APPLICATION

(11) **EP 4 494 638 A1**
(43) Date of publication of application: **22.01.2025**
(21) Application number: 23770828.4
(22) Date of filing: 15.03.2023
(51) Int. Cl.: A61K 31/09, A23L 3/349, A23L 3/3517, A23L 3/3544, A23L 3/3553, A23L 33/10, A61K 9/14, A61K 47/14, A61K 47/22, A61K 47/24, A61K 47/38, A61P 3/00, A61P 9/00, A61P 39/06, A61P 43/00

(54) **METHOD FOR PRESERVING SOLID COMPOSITION THAT CONTAINS FORM II TYPE CRYSTALS OF REDUCED COENZYME Q10, AND PACKING THAT CONTAINS SAID SOLID COMPOSITION**

(30) Priority: 17.03.2022 JP 2022042288
(71) Applicant: Kaneka Corporation, Osaka-shi, Osaka 530-8288 (JP)
(72) Inventor: FUKUYAMA Yuka, Takasago-shi, Hyogo 676-8688 (JP); KITAMURA Shiro, Osaka-shi, Osaka 530-8288 (JP); YOKOCHI Yuichi, Takasago-shi, Hyogo 676-8688 (JP)
(74) Representative: Hoffmann Eitle
(86) International application number: PCT/JP2023/010053
(87) International publication number: WO 2023/176875

(57) **Abstract**

The present specification discloses a means capable of controlling the decrease of reduced coenzyme Q10 due to its oxidation during storage of a solid composition containing the reduced coenzyme Q10. One or more embodiments of the present invention relate to a method for storing a solid composition containing a reduced coenzyme Q10 Form II crystal, an emulsifier having an HLB of 6.0 or more, and an antioxidant, the method including storing the solid composition in a gas phase having a relative humidity of less than 50%. One or more embodiments of the present invention relate to a package including: a solid composition containing a reduced coenzyme Q10 Form II crystal, an emulsifier having an HLB of 6.0 or more, and an antioxidant; a gas phase having a relative humidity of less than 50%; and a container enclosing the solid composition and the gas phase.

## Description

### Technical Field

One or more embodiments of the present invention relate to a method for storing a solid composition containing a reduced coenzyme Q10 Form II crystal.

Another one or more embodiments of the present invention relate to a package containing a solid composition containing a reduced coenzyme Q10 Form II crystal.

### Background Art

Coenzyme Q is an essential component widely distributed in living organisms from bacteria to mammals, and is known as a member of the mitochondrial electron transfer system in cells in living organisms. In humans, the major form of coenzyme Q is coenzyme Q10 the side chain of which has ten repeating structures. In the living body, approximately 40% to 90% of the coenzyme Q10 is generally present in reduced form. The physiological activity of coenzyme Q includes activation of energy production by mitochondrial activation, activation of cardiac function, stabilization of cell membranes, and protection of cells by antioxidant activity.

While coenzyme Q10 currently produced and sold is, in large part, oxidized coenzyme Q10, reduced coenzyme Q10 (hereinafter sometimes referred to as "QH") which exhibits higher oral absorbability than oxidized coenzyme Q10 has also been commercially available and has come to be used in recent years.

Reduced coenzyme Q10 is easily oxidized, and therefore, has the problems of high storage costs, and limited scope of application to commercial forms.

Patent Literature 1 discloses that crystal polymorphism is found in reduced coenzyme Q10, and that a newly appearing crystalline form (this crystal is hereinafter referred to as a "reduced coenzyme Q10 Form II crystal", "QH Form II crystal", or "Form II crystal") is much more stable than the conventional reduced coenzyme Q10 (this crystal is hereinafter referred to as a "reduced coenzyme Q10 Form I crystal", "QH Form I crystal", or "Form I crystal"), and also has other excellent physical properties.

Patent Literature 2 describes a reduced coenzyme Q10 composition that is suited for an inexpensive simple formulation capable of meeting market demand, has good oxidation stability, and is in a solid state such as a powder at room temperature, wherein the composition contains reduced coenzyme Q10, an organic acid, and a carbonate containing a sodium cation and/or a calcium cation, and is solid at 25°C. The Patent Literature 2 discloses that the reduced coenzyme Q10 is preferably a Form II crystal. Patent Literature 2 discloses that, to improve the oxidation stability of the reduced coenzyme Q10 in the composition, it is essential to incorporate a carbonate containing a sodium cation and/or a calcium cation.

Patent Literature 3 describes a composition that contains reduced coenzyme Q10 and is stable to oxidation, wherein the composition is a particulate composition in which an oily component (A) containing reduced coenzyme Q10 and a lipophilic antioxidant is polydispersed in domain form in a matrix containing a water-soluble excipient as a main component. Patent Literature 3 further discloses that the water-soluble excipient may contain a surfactant, that the surfactant is preferably hydrophilic, and has, for example, an HLB of 4 or more, typically an HLB of 6 or more, preferably an HLB of 8 or more, that the oily component (A) preferably further contains a lipophilic surfactant, and that a surfactant which has, for example, an HLB of 10 or less, preferably 8 or less, more preferably 6 or less, still more preferably 5 or less is usable. Patent Literature 3 discloses that, in the particulate composition, 10% by weight or more, preferably 20% by weight or more, more preferably 50% by weight or more, still more preferably 70% by weight or more, particularly preferably 80% by weight or more, of the reduced coenzyme Q10 is preferably not in a crystalline state but in an amorphous or melt state.

### Citation List

### Patent Literature

Patent Literature 1: WO 2012/176842
Patent Literature 2: WO 2015/122531
Patent Literature 3: JP 2009-149584 A

### Summary of Invention

### Technical Problem

As described above, means for preventing the oxidation of reduced coenzyme Q10 have been developed so far. However, the invention described in Patent Literature 2 requires the addition of the specific component, i.e., a carbonate containing a sodium cation and/or a calcium cation to stabilize reduced coenzyme Q10. The invention described in Patent Literature 3 requires the formulation of reduced coenzyme Q10 into a specific form. Such requirements restrict the form and application of a composition containing reduced coenzyme Q10. Therefore, there is still a demand for a new means for preventing the oxidation of reduced coenzyme Q10.

In view of the above, the present specification discloses a novel means capable of controlling the decrease of reduced coenzyme Q10 due to its oxidation during storage of a solid composition containing the reduced coenzyme Q10.

### Solution to Problem

The present inventors have completed each of the below-described embodiments of the present invention through the new discovery that, while a solid composition containing a reduced coenzyme Q10 Form II crystal together with an emulsifier and an antioxidant is in storage, the decrease of the reduced coenzyme Q10 in the solid composition due to its oxidation is controlled.
(1) A method for storing a solid composition containing a reduced coenzyme Q10 Form II crystal, an emulsifier having an HLB of 6.0 or more, and an antioxidant, the method including storing the solid composition in a gas phase having a relative humidity of less than 50%.
(2) The method according to (1), wherein the gas phase is air.
(3) The method according to (1) or (2), wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.
(4) The method according to any one of (1) to (3), wherein the antioxidant is one or more selected from the group consisting of ascorbic acid, ascorbic acid salts, erythorbic acid, and erythorbic acid salts.
(5) The method according to any one of (1) to (4), wherein the solid composition contains 1 part by weight or more and 9900 parts by weight or less of the emulsifier with respect to 100 parts by weight of the crystal.
(6) The method according to any one of (1) to (5), wherein the amount of the emulsifier contained in the solid composition is 1% by weight or more and 99% by weight or less.
(7) The method according to any one of (1) to (6), wherein the solid composition further contains a binder.
(8) The method according to (7), wherein the binder is one or more selected from the group consisting of hydroxypropyl cellulose and hydroxypropylmethyl cellulose.
(9) The method according to (7) or (8), wherein the solid composition is a particulate solid composition.
(10) The method according to any one of (1) to (9), wherein the solid composition is free of a carbonate containing a sodium cation and/or a calcium cation.
(11) The method according to any one of (1) to (10), wherein the solid composition is not a composition in which an oily component containing reduced coenzyme Q10 and a lipophilic antioxidant is polydispersed in domain form in a matrix containing a water-soluble excipient.
(12) A package including:
   a solid composition containing a reduced coenzyme Q10 Form II crystal, an emulsifier having an HLB of 6.0 or more, and an antioxidant;
   a gas phase having a relative humidity of less than 50%; and
   a container enclosing the solid composition and the gas phase.
(13) The package according to (12), wherein the gas phase is air.
(14) The package according to (12) or (13), wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.
(15) The package according to any one of (12) to (14), wherein the antioxidant is one or more selected from the group consisting of ascorbic acid, ascorbic acid salts, erythorbic acid, and erythorbic acid salts.
(16) The package according to any one of (12) to (15), wherein the solid composition contains 1 part by weight or more and 9900 parts by weight or less of the emulsifier with respect to 100 parts by weight of the crystal.
(17) The package according to any one of (12) to (16), wherein the amount of the emulsifier contained in the solid composition is 1% by weight or more and 99% by weight or less.
(18) The package according to any one of (12) to (17), wherein the solid composition further contains a binder.
(19) The package according to (18), wherein the binder is one or more selected from the group consisting of hydroxypropyl cellulose and hydroxypropylmethyl cellulose.
(20) The package according to (18) or (19), wherein the solid composition is a particulate solid composition.
(21) The package according to any one of (12) to (20), wherein the solid composition is free of a carbonate containing a sodium cation and/or a calcium cation.
(22) The package according to any one of (12) to (21), wherein the solid composition is not a composition in which an oily component containing reduced coenzyme Q10 and a lipophilic antioxidant is polydispersed in domain form in a matrix containing a water-soluble excipient.

The present specification encompasses the disclosure of Japanese Patent Application No. 2022-042288 that serves as a basis for the priority of the present application.

### Advantageous Effects of Invention

The method according to one or more embodiments of the present invention allows control of the decrease of reduced coenzyme Q10 due to its oxidation during the storage of the solid composition containing the reduced coenzyme Q10 Form II crystal.

The package according to another one or more embodiments of the present invention allows control of the decrease of reduced coenzyme Q10 due to its oxidation during the storage of the solid mixture containing the reduced coenzyme Q10 Form II crystal.

### Description of Embodiments

Hereinafter, the present invention will be described in detail.

### <Reduced Coenzyme Q10>

The reduced coenzyme Q10 herein may partially include oxidized coenzyme Q10 as long as the reduced coenzyme Q10 is included as a main component. The main component herein means that it is included in a percentage of, for example, 50% by weight or more, typically 60% by weight or more, preferably 70% by weight or more, more preferably 80% by weight or more, still more preferably 90% by weight or more, particularly preferably 95% by weight or more, and particularly 98% by weight or more. The above-described percentages are the percentages of the reduced coenzyme Q10 to the total weight of coenzyme Q10.

As described above, the reduced coenzyme Q10 encompasses two forms of crystal polymorphisms, i.e., Form I and Form II. Specifically, the crystalline form of reduced coenzyme Q10 having a melting point around 48°C and showing characteristic peaks at diffraction angles (2θ ± 0.2°) of 3.1°, 18.7°, 19.0°, 20.2°, and 23.0° in powder X-ray (Cu-Kα) diffraction is a Form I crystal, whereas the crystalline form of reduced coenzyme Q10 having a melting point around 52°C and showing characteristic peaks at diffraction angles (2θ ± 0.2°) of 11.5°, 18.2°, 19.3°, 22.3°, 23.0°, and 33.3° in powder X-ray (Cu-Kα) diffraction is a Form II crystal.

One or more embodiments of the present invention are characterized by using reduced coenzyme Q10 (QH) in the form of a reduced coenzyme Q10 Form II crystal (QH Form II crystal). The QH Form II crystal has high stability to oxidation, and thus, a solid composition containing the QH Form II crystal together with an emulsifier and an antioxidant has excellent storage stability.

The QH Form II crystal may be composed of a Form II crystal alone, or may be a QH crystal or crystalline solid containing a Form II crystal as a main component. Here, the main component means that the amount of the Form II crystal is preferably 80% by weight or more, more preferably 90% by weight or more, more preferably 95% by weight or more, most preferably 98% by weight or more, with respect to the total amount of QH.

### <Emulsifier>

Herein, an emulsifier having an HLB of 6.0 or more is used. The emulsifier is not limited to any kind. Two or more kinds of emulsifiers may be used in combination. The HLB of the emulsifier is more preferably 7.0 or more. The upper limit of the HLB of the emulsifier is not particularly limited, and is preferably 16.0 or less, more preferably 15.5 or less.

Specific examples of the emulsifier include one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.

The number of glycerol units in the polyglycerol may be 2 or more, and is preferably 2 or more and 10 or less. Examples include diglycerol, triglycerol, tetraglycerol, pentaglycerol, hexaglycerol, and decaglycerol.

The number of oxyethylene units in the polyoxyethylene sorbitan may be 2 or more, preferably 10 or more and 30 or less, more preferably 15 or more and 25 or less.

The number of propylene glycol units in the polypropylene glycol may be 2 or more, and is preferably 2 or more and 10 or less.

The number of ethylene glycol units in the polyethylene glycol may be 2 or more, and is preferably 2 or more and 10 or less.

The polyol is particularly preferably one or more selected from the group consisting of monoglycerol, polyglycerol, and sucrose.

Examples of the fatty acid optionally having a substituent include linear or branched, monovalent or divalent fatty acids having 4 or more and 24 or less carbon atoms. Examples of the substituent include a hydroxyl group and an acetoxy group. The number of the substituents is preferably 2 or less. Specific examples of the fatty acid optionally having a substituent include lauric acid, oleic acid, caprylic acid, stearic acid, behenic acid, ricinoleic acid, succinic acid, and diacetyl tartaric acid. One or more selected from the group consisting of lauric acid, stearic acid, caprylic acid, oleic acid, and succinic acid are particularly preferable.

In the ester compound of the polyol and the fatty acid, the number of fatty acids bound to one polyol molecule is not particularly limited, and may be appropriately adjusted in accordance with the intended HLB of the emulsifier.

Specific examples of the ester compound of the polyol and the fatty acid include diglycerol monooleate, monoglycerol monocaprylate, diglycerol monocaprylate, decaglycerol pentaoleate, tetraglycerol pentaoleate, pentaglycerol trioleate, decaglycerol monolaurate, hexaglycerol monocaprylate, hexaglycerol monooleate, pentaglycerol monostearate, tetraglycerol tristearate, decaglycerol monobehenate, mono- and di-glycerol monostearates, monoglycerol monooleate, glycerol monostearate succinate, monoglycerol succinate, glycerol monostearate diacetyl tartrate, propylene glycol monooleate, sorbitan monooleate, sorbitan tristearate, diglycerol monolaurate, diglycerol monomyristate, polyoxyethylene sorbitan monooleate, polyoxyethylene sorbitan monolaurate, polyoxyethylene sorbitan monostearate, condensed pentaglycerol ricinoleate, sucrose stearate, sucrose erucate, and sucrose oleate. Specific examples of a more preferable ester compound of the polyol and the fatty acid include diglycerol monooleate, diglycerol monocaprylate, pentaglycerol trioleate, decaglycerol monolaurate, hexaglycerol monocaprylate, hexaglycerol monooleate, pentaglycerol monostearate, glycerol monostearate succinate, and sucrose stearate. Particularly preferable specific examples of the ester compound of the polyol and the fatty acid include diglycerol monooleate.

Examples of the lecithin include soybean lecithin, egg yolk lecithin, and enzymolytic soybean lecithin.

The emulsifier to be used is particularly preferably an emulsifier acceptable for foods, cosmetics, and/or pharmaceutical products.

### <Antioxidant>

Herein, the antioxidant is not limited to any kind. Two or more kinds of antioxidants may be used in combination. Specific examples of the antioxidant include one or more selected from the group consisting of ascorbic acid, ascorbic acid salts, erythorbic acid, and erythorbic acid salts. The antioxidant is particularly preferably free of a lipophilic antioxidant such as an ascorbyl fatty acid ester.

The counterion of each of the ascorbic acid salt and the erythorbic acid salt is not limited, and the ascorbic acid salt and the erythorbic acid salt may be independently one or more metal salts selected from the group consisting of sodium salts, potassium salts, calcium salts, and magnesium salts.

The antioxidant to be used is particularly preferably an antioxidant acceptable for foods or pharmaceutical products. The antioxidant acceptable for foods or pharmaceutical products is preferably an ascorbic acid salt, particularly preferably one or more selected from the group consisting of sodium ascorbate and calcium ascorbate.

### <Binder>

The solid composition disclosed herein preferably further contains a binder. The binder can be used for binding components including the reduced coenzyme Q10 Form II crystal, the emulsifier having an HLB of 6.0 or more, and the antioxidant, to form a particulate solid composition.

The binder is not limited to any kind. Two or more kinds of binders may be used in combination. Specific examples of the binder include one or more selected from the group consisting of celluloses and starches.

Examples of the celluloses include hydroxypropyl cellulose, hydroxypropylmethyl cellulose, hydroxymethyl cellulose, carboxymethyl cellulose, crystalline cellulose, powdered cellulose, methyl cellulose, ethyl cellulose, and salts thereof. The binder is particularly preferably one or more selected from the group consisting of hydroxypropyl cellulose, hydroxypropylmethyl cellulose, and sodium carboxymethyl cellulose, still more preferably one or more selected from the group consisting of hydroxypropyl cellulose and hydroxypropylmethyl cellulose.

Examples of the starches include wheat starch, potato starch, sweet potato starch, corn starch, dextrin, hydroxypropyl starch, starch acetate, oxidized starch, octenyl succinate starch and salts thereof, and partially pregelatinized starch.

The binder to be used is particularly preferably a binder acceptable for foods or pharmaceutical products.

### <Solid Composition>

The solid composition used in one or more embodiments of the present invention includes the reduced coenzyme Q10 Form II crystal, the emulsifier having an HLB of 6.0 or more, and the antioxidant.

When stored in a gas phase having a relative humidity of less than 50%, the solid composition allows to control the decrease of the reduced coenzyme Q10 due to oxidation, and thus, can be stored for a long time, for example, a period of three months or more.

The solid composition is a composition that is solid at 25°C. The solid composition may have any shape, such as a particulate, powdery, or flaky shape, and preferably has a particulate shape. The particulate shape may be any shape formed by binding primary particles of a material containing the reduced coenzyme Q10 Form II crystal, the emulsifier having an HLB of 6.0 or more, and the antioxidant by granulation, and encompasses a granular shape. The particulate solid composition is not limited to any dimensions, and can be, for example, granules having a longest diameter of 0.20 mm or more and 2.0 mm or less.

In the solid composition, the ratio of each component can be adjusted so that the oxidation of the reduced coenzyme Q10 can be controlled.

The solid composition more preferably contains the antioxidant, for example, at 1 part by weight or more and 9900 parts by weight or less with respect to 100 parts by weight of the reduced coenzyme Q10 Form II crystal. The lower limit of the amount of the antioxidant with respect to 100 parts by weight of the reduced coenzyme Q10 Form II crystal is preferably 20 parts by weight or more, more preferably 50 parts by weight or more, more preferably 80 parts by weight or more. The upper limit of the amount of the antioxidant with respect to 100 parts by weight of the reduced coenzyme Q10 Form II crystal is preferably 5000 parts by weight or less, more preferably 1000 parts by weight or less, more preferably 500 parts by weight or less, more preferably 200 parts by weight or less, more preferably 120 parts by weight or less.

The solid composition more preferably contains the emulsifier having an HLB of 6.0 or more, for example, at 1 part by weight or more and 9900 parts by weight or less with respect to 100 parts by weight of the reduced coenzyme Q10 Form II crystal. The lower limit of the amount of the emulsifier with respect to 100 parts by weight of the reduced coenzyme Q10 Form II crystal is preferably 3 parts by weight or more, more preferably 5 parts by weight or more, more preferably 8 parts by weight or more. The upper limit of the amount of the emulsifier with respect to 100 parts by weight of the reduced coenzyme Q 10 Form II crystal is preferably 5000 parts by weight or less, more preferably 1000 parts by weight or less, more preferably 500 parts by weight or less, more preferably 200 parts by weight or less, more preferably 150 parts by weight or less, more preferably 100 parts by weight or less, more preferably 50 parts by weight or less, more preferably 30 parts by weight or less.

The lower limit of the amount of the reduced coenzyme Q10 Form II crystal contained in the solid composition is, for example, 1% by weight or more, preferably 10% by weight or more, more preferably 20% by weight or more, more preferably 30% by weight or more. The upper limit of the amount of the reduced coenzyme Q10 Form II crystal is, for example, 90% by weight or less, preferably 80% by weight or less, more preferably 70% by weight or less, more preferably 60% by weight or less.

The lower limit of the amount of the antioxidant contained in the solid composition is, for example, 1% by weight or more, preferably 10% by weight or more, more preferably 20% by weight or more, more preferably 30% by weight or more. The upper limit of the amount of the antioxidant is, for example, 99% by weight or less, preferably 90% by weight or less, preferably 80% by weight or less, more preferably 70% by weight or less, more preferably 60% by weight or less.

The lower limit of the amount of the emulsifier having an HLB of 6.0 or more contained in the solid composition is, for example, 0.5% by weight or more, preferably 1% by weight or more, more preferably 3% by weight or more. The upper limit of the amount of the emulsifier having an HLB of 6.0 or more is, for example, 99% by weight or less, preferably 50% by weight or less, more preferably 20% by weight or less, more preferably 15% by weight or less, more preferably 10% by weight or less.

When the solid composition contains the binder, the lower limit of the amount of the binder contained in the solid composition is, for example, 1% by weight or more, preferably 5% by weight or more, more preferably 8% by weight or more. The upper limit of the amount of the binder is, for example, 20% by weight or less, preferably 15% by weight or less, more preferably 10% by weight.

The solid composition more preferably contains the binder, for example, at an amount of 1 part by weight or more, preferably 3 parts by weight or more, more preferably 5 parts by weight or more, more preferably 8 parts by weight or more, with respect to 100 parts by weight of the reduced coenzyme Q10 Form II crystal. In addition, the solid composition contains the antioxidant, for example, at an amount of 200 parts by weight or less, preferably 150 parts by weight or less, more preferably 100 parts by weight or less, more preferably 50 parts by weight or less, still more preferably 30 parts by weight or less, with respect to 100 parts by weight of the reduced coenzyme Q10 Form II crystal.

The solid composition may further contain another component such as an excipient. Another component is preferably a component acceptable for foods or pharmaceutical products.

The solid composition is preferably free of a carbonate containing a sodium cation and/or a calcium cation. Examples of the carbonate containing a sodium cation include sodium carbonate and hydrates thereof (for example, monohydrates, heptahydrates, decahydrates, and the like) and sodium bicarbonate. Examples of the carbonate containing a calcium cation include calcium carbonate and hydrates thereof (for example, 0.65-hydrates, monohydrates, 1.5-hydrates, hexahydrates, and the like). Storing the solid composition by a method according to one or more embodiments of the present invention allows to control the oxidation of reduced coenzyme Q10 without adding a specific component, i.e., a carbonate containing a sodium cation and/or a calcium cation, to the solid composition.

The solid composition is preferably not a composition in which an oily component containing reduced coenzyme Q10 and a lipophilic antioxidant is polydispersed in domain form in a matrix containing a water-soluble excipient. Storing the solid composition by a method according to one or more embodiments of the present invention allows to control the oxidation of reduced coenzyme Q10 without the need for the solid composition to have a particular multiphase structure.

### <Method for Storage>

One or more embodiments of the present invention relate to a method for storing the solid composition containing the reduced coenzyme Q10 Form II crystal, the emulsifier having an HLB of 6.0 or more, and the antioxidant, the method including storing the solid composition in a gas phase having a relative humidity of less than 50%.

The method according to the present embodiment is based on the unexpected discovery that the oxidation of the reduced coenzyme Q10 is controlled when the solid composition is stored in a gas phase having a relative humidity of less than 50%, as compared with when the solid composition is stored in a gas phase having a relative humidity of 50% or more. In a preferable aspect of the method according to the present embodiment, the reduced coenzyme Q10 can be maintained in an amount of 90% by weight or more of the initial amount for a period as long as two months or more even under a condition of a temperature as high as 40°C. In order to provide the solid composition in the above-described form at a reasonable price, it is preferable that the reduced coenzyme Q10 can be maintained in an amount of 90% by weight or more, preferably 93% by weight or more, more preferably 95% by weight or more, of the initial amount at 40°C for two months.

In the method according to the present embodiment, the relative humidity of the gas phase is, for example, less than 50%, preferably 45% or less, more preferably 40% or less, more preferably 30% or less, more preferably 20% or less, more preferably 15% or less, and preferably 1% or more, more preferably 5% or more.

The temperature at which the solid composition is stored in the method according to the present embodiment can be, for example, a temperature of -25°C to 50°C, preferably a temperature of -20°C or more, -10°C or more, 0°C or more, 4°C or more, 5°C or more, 10°C or more, 15°C or more, or 20°C or more, and preferably a temperature of 45°C or less, or 40°C or less. The temperature can be specifically 10°C, 15°C, 25°C or 40°C. In the method according to the present embodiment, the relative humidity of the gas phase is the relative humidity of the gas phase at a storage temperature.

The period for which the solid composition is stored in the method according to the present embodiment is not particularly limited, as long as it is a period from production to use of a product, and can be appropriately adjusted depending on storage conditions such as temperature. The period can be preferably 3 days or more, 1 week or more, or 2 weeks or more, and, for example, 5 years or less, typically 3 years or less, preferably 2 years or less, more preferably 1 year or less, more preferably 6 months or less, more preferably 8 weeks or less, and more preferably 6 weeks or less, more preferably 5 weeks or less, more preferably 4 weeks or less.

In the method according to the present embodiment, the gas phase is preferably air. The method using air as the gas phase is preferable because the method can be performed at a low cost, compared with a method using an inert gas such as nitrogen as the gas phase.

### <Package>

Another one or more embodiments of the present invention relate to a package including:
a solid composition containing a reduced coenzyme Q10 Form II crystal, an emulsifier having an HLB of 6.0 or more, and an antioxidant;
a gas phase having a relative humidity of less than 50%; and
a container enclosing the solid composition and the gas phase.

The package according to the present embodiment allows control of the decrease of the reduced coenzyme Q10 due to its oxidation during storage, and is therefore preferable.

The container is not particularly limited as long as it is capable of containing the solid composition together with a gas phase and being hermetically sealed. The container may be, for example, a hermetically sealable glass container, metal container, resin container, wooden container, or bag.

In the package according to the present embodiment, the relative humidity of the gas phase is, for example, less than 50%, preferably 45% or less, more preferably 40% or less, more preferably 30% or less, more preferably 20% or less, more preferably 15% or less, and preferably 1% or more, more preferably 5% or more.

In the package according to the present embodiment, the relative humidity of the gas phase is the relative humidity of the gas phase at a temperature at which the package is stored. The temperature at which the package is stored can be, for example, a temperature of -25°C to 50°C, preferably a temperature of -20°C or more, -10°C or more, 0°C or more, 4°C or more, 5°C or more, 10°C or more, 15°C or more, or 20°C or more, and preferably a temperature of 45°C or less, or 40°C or less. The temperature can be specifically 10°C, 15°C, 25°C or 40°C.

In the package according to the present embodiment, the gas phase is preferably air. The method using air as the gas phase is preferable because the method can be performed at a low cost, compared with a method using an inert gas such as nitrogen as the gas phase.

### Examples

The present invention will be further specifically described with reference to the following Examples, but the present invention is not limited to these Examples. It should be noted that the HPLC measurement conditions in the following experiment are as described below.

### (Method for Evaluating Oxidation Stability)

The weight ratio of the reduced coenzyme Q10 to the total coenzyme Q10 (i.e., reduced coenzyme Q10/(oxidized coenzyme Q10 + reduced coenzyme Q10)) is defined as a "QH ratio". The QH ratio was determined by the following HPLC analysis. For the evaluation of oxidation stability, a rate of the change in the QH ratio at the end of the evaluation from that at the beginning of the evaluation was defined as "QH residual rate", and the QH residual rate determined by the following formula was used as a measure of oxidation stability. QH residual rate (%) = 100 × QH ratio at the end of the evaluation/QH ratio at the beginning of the evaluation

### (HPLC Measurement Conditions)

Column: SYMMETRY C₁₈ (manufactured by Waters Corporation); 250 mm (in length) and 4.6 mm (in inner diameter)
Mobile phase: C₂H₅OH:CH₃OH = 4:3 (v:v)
Detection wavelength: 210 nm
Flow rate: 1 mL/min

### <Example 1>

### (Experiment)

A reduced coenzyme Q10 Form II crystal in an amount of 1.0 g, 1.0 g of sodium ascorbate, 0.2 g of hydroxypropyl cellulose, and 0.2 g of any of the emulsifiers (A1 to A9) listed in Table 1 were mixed, and water was added. The resulting mixture was kneaded. The kneaded material obtained was extrusion-granulated using a 1.2 mm Φ screen, and dried to obtain a particulate solid composition. The resulting particulate solid composition was stored in air at a temperature of 40°C and a relative humidity of 75% or a temperature of 40°C and a relative humidity of 10% for two months, and the storage stability of the reduced coenzyme Q10 was evaluated on the basis of the QH residual rate.

**[Table 1]**

| No. | Name | Product Name | HLB Value |
|---|---|---|---|
| A1 | decaglycerol monolaurate | SUNSOFT M-12J | 15.5 |
| | | manufactured by Taiyo Kagaku Co., Ltd. | |
| A2 | sucrose stearate | Ryoto Sugar Ester S-1570 | 15 |
| | | manufactured by Mitsubishi Chemical Corporation | |
| A3 | pentaglycerol monostearate | SUNSOFT A-181E | 13 |
| | | manufactured by Taiyo Kagaku Co., Ltd. | |
| A4 | hexaglycerol monooleate | SY-Glyster MO-5S | 11.6 |
| | | manufactured by Sakamoto Yakuhin | |
| | | Kogyo Co., Ltd. | |
| A5 | diglycerol monocaprylate | SUNSOFT Q-10D | 9.5 |
| | | manufactured by Taiyo Kagaku Co., Ltd. | |
| A6 | glycerol monostearate succinate | SUNSOFT No.681SPV | 8.5 |
| | | manufactured by Taiyo Kagaku Co., Ltd. | |
| A7 | diglycerol monooleate | SUNSOFT Q-17D | 7.5 |
| | | manufactured by Taiyo Kagaku Co., Ltd. | |
| A8 | diglycerol monooleate | POEM DO-100V | 7.4 |
| | | Riken Vitamin Co., Ltd. | |
| A9 | pentaglycerol trioleate | SUNSOFT A-173E | 7 |
| | | manufactured by Taiyo Kagaku Co., Ltd. | |

### (Results)

The QH residual rates of the particulate solid compositions after being stored at each humidity in the above-described experiment are shown in the following Table.

**[Table 2]**

| | QH Residual Rate (%) | |
|---|---|---|
| No. | Relative Humidity of 75% | Relative Humidity of 10% |
| A1 | 92.1 | 97.6 |
| A2 | 91.6 | 96.9 |
| A3 | 90.8 | 96.9 |
| A4 | 91.7 | 95.7 |
| A5 | 90.7 | 93.6 |
| A6 | 83.2 | 95.4 |
| A7 | 86.9 | 98.0 |
| A8 | 89.6 | 95.5 |
| A9 | 91.6 | 95.8 |

### <Example 2>

A reduced coenzyme Q10 Form II crystal in an amount of 150 g, 135 g of pulverized sodium ascorbate, and 15 g of diglycerol monooleate (SUNSOFT Q-17D) were mixed. The mixture obtained was introduced into a high-shear granulator (SPGJ-2TG, manufactured by Dalton Corporation) and mixed, and then, 20 mL of water was added. The resulting mixture was granulated, and dried to obtain a particulate solid composition. The resulting particulate solid composition was stored in air at a temperature of 40°C and a relative humidity of 75% or a temperature of 40°C and a relative humidity of 10% for two months, and the storage stability of the reduced coenzyme Q10 was evaluated on the basis of the QH residual rate.

### <Example 3>

A reduced coenzyme Q10 Form II crystal in an amount of 275 g, 165 g of pulverized sodium ascorbate, 25 g of diglycerol monooleate (SUNSOFT Q-17D), and 35 g of hydroxypropyl cellulose were mixed. The mixture obtained was introduced into a high-shear granulator and mixed, and then, 15 mL of water was added. The resulting mixture was granulated, and dried to obtain a particulate solid composition. The resulting particulate solid composition was stored in air at a temperature of 40°C and a relative humidity of 75% or a temperature of 40°C and a relative humidity of 10% for two months, and the storage stability of the reduced coenzyme Q10 was evaluated on the basis of the QH residual rate.

### <Example 4>

A reduced coenzyme Q10 Form II crystal in an amount of 326 g, 149 g of pulverized sodium ascorbate, and 25 g of diglycerol monooleate (SUNSOFT Q-17D) were mixed. The mixture obtained was introduced into a high-shear granulator and mixed, and then, 15 mL of water was added. The resulting mixture was granulated, and dried to obtain a particulate solid composition. The resulting particulate solid composition was stored in air at a temperature of 40°C and a relative humidity of 75% or a temperature of 40°C and a relative humidity of 10% for two months, and the storage stability of the reduced coenzyme Q10 was evaluated on the basis of the QH residual rate.

The results obtained in Examples 2 to 4 are shown in Table 3.

**[Table 3]**

| | QH Residual Rate (%) | |
|---|---|---|
| | Relative Humidity of 75% | Relative Humidity of 10% |
| Example 2 | 91.1 | 94.9 |
| Example 3 | 90.8 | 95.9 |
| Example 4 | 91.5 | 98.0 |

The results in Table 2 and Table 3 indicate that the particulate solid composition containing the antioxidant and the emulsifier having a high HLB value together with the reduced coenzyme Q10 Form II crystal has higher storage stability under low-humidity conditions.

### <Example 5>

### (Experiment)

Using each of the salts shown in Table 4, approximately 50 ml of a saturated aqueous solution was prepared. Each of the salts was used in excess of its solubility, leaving the solid salt in the solution so that the salt concentration of the aqueous solution could not be changed by moisture absorption. Each of the saturated aqueous solutions containing the solid salt was divided into two dishes having a diameter of 90 mm. Both of the two dishes were placed together in a polycarbonate jar (having an internal volume of 7000 ml), and this procedure was repeated for each salt to obtain packages (1) and (2). The relative humidity in the packages (1) and (2) was determined in accordance with Greenspan, *J Res NBS A Phys Ch,* 1977.

**[Table 4]**

| Package No. | Salt Used | Relative Humidity (%) |
|---|---|---|
| (1) | magnesium chloride | 32 |
| (2) | potassium iodide | 66 |

The particulate solid compositions of Example 1 A1 and Example 1 A7 were packed together in both of the packages (1) and (2) shown in Table 4 in an opened state, and then the packages were hermetically sealed. In each of the packages, the saturated aqueous salt solution and the particulate solid compositions were separated from each other. The packages containing the particulate solid compositions were stored at 40°C for three months, and then the QH residual rates were determined.

### (Results)

The QH residual rates after storage at 40°C for three months in Example 5, Example 1 A1, and Example 1 A7 are shown in Tables 5 and 6. In Example 5, the particulate solid compositions of Example 1 A1 and Example 1 A7 that were each packed in the package (1) were referred to as "Example 5-(1) A1" and "Example 5-(1) A7" respectively, and the particulate solid compositions of Example 1 A1 and Example 1 A7 that were each packed in the package (2) were referred to as "Example 5-(2) A1" and "Example 5-(2) A7" respectively.

**[Table 5]**

| No. | Relative Humidity (%) | QH Residual Rate (%) |
|---|---|---|
| Example 1 A1 | 10 | 96.7 |
| Example 5-(1) A1 | 32 | 96.7 |
| Example 5-(2) A1 | 66 | 91.9 |
| Example 1 A1 | 75 | 82.3 |

**[Table 6]**

| No. | Relative Humidity (%) | QH Residual Rate (%) |
|---|---|---|
| Example 1 A7 | 10 | 95.3 |
| Example 5-(1) A7 | 32 | 97.3 |
| Example 5-(2) A7 | 66 | 92.5 |
| Example 1 A7 | 75 | 73.2 |

The results shown in Table 5 and Table 6 indicate that the particulate solid composition containing the antioxidant and the emulsifier having an HLB value of 6.0 or more together with the reduced coenzyme Q10 Form II crystal has higher storage stability under conditions of low humidity of less than 50%.

### <Reference Example 1>

### (Experiment)

A reduced coenzyme Q10 Form II crystal in an amount of 1.0 g, 1.0 g of sodium ascorbate, 0.2 g of hydroxypropyl cellulose, and 0.2 g of glycerol monostearate succinate (POEM B-10, manufactured by Riken Vitamin Co., Ltd.; having an HLB value of 5) were mixed, and water was added. The resulting mixture was kneaded. The kneaded material obtained was extrusion-granulated using a 1.2 mm Φ screen, and dried to obtain a particulate solid composition. The resulting particulate solid composition was stored in air at a temperature of 40°C and a relative humidity of 10% for two months, and the storage stability of the reduced coenzyme Q10 was evaluated on the basis of the QH residual rate.

### (Results)

The QH residual rate after two months of storage under the above-described low-humidity condition was 90.0%, which was lower than the QH residual rate when the emulsifier having an HLB value of 6.0 or more was used.

All publications, patents, and patent applications cited herein are incorporated herein by reference in their entirety.

The upper and/or lower limits of the numerical ranges herein may be arbitrarily combined to define a preferred range. For example, any upper limit and any lower limit of the numerical ranges may be combined to define a preferred range. Any upper limits of the numerical ranges may be combined to define a preferred range. Any lower limits of the numerical ranges may be combined to define a preferred range. A numerical range described herein with the term "to" includes the values presented before and after the term "to" as a lower limit and an upper limit, respectively.

It should be understood that throughout the present description, expressions in the singular include the plural, unless otherwise specified. Accordingly, the singular articles (e.g., "a", "an", and "the" in English) include plural references unless otherwise specified.

Although, the present embodiments are described in detail above, the specific configuration is not limited to these embodiments, and the present disclosure encompasses any design modification within the scope which does not depart from the spirit of the present disclosure.

## Claims

1. A method for storing a solid composition comprising a reduced coenzyme Q10 Form II crystal, an emulsifier having an HLB of 6.0 or more, and an antioxidant, the method comprising
storing the solid composition in a gas phase having a relative humidity of less than 50%.

2. The method according to claim 1, wherein the gas phase is air.

3. The method according to claim 1 or 2, wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.

4. The method according to any one of claims 1 to 3, wherein the antioxidant is one or more selected from the group consisting of ascorbic acid, ascorbic acid salts, erythorbic acid, and erythorbic acid salts.

5. The method according to any one of claims 1 to 4, wherein the solid composition comprises 1 part by weight or more and 9900 parts by weight or less of the emulsifier with respect to 100 parts by weight of the crystal.

6. The method according to any one of claims 1 to 5, wherein the amount of the emulsifier contained in the solid composition is 1% by weight or more and 99% by weight or less.

7. The method according to any one of claims 1 to 6, wherein the solid composition further comprises a binder.

8. The method according to claim 7, wherein the binder is one or more selected from the group consisting of hydroxypropyl cellulose and hydroxypropylmethyl cellulose.

9. The method according to claim 7 or 8, wherein the solid composition is a particulate solid composition.

10. The method according to any one of claims 1 to 9, wherein the solid composition is free of a carbonate containing a sodium cation and/or a calcium cation.

11. The method according to any one of claims 1 to 10, wherein the solid composition is not a composition in which an oily component containing reduced coenzyme Q10 and a lipophilic antioxidant is polydispersed in domain form in a matrix containing a water-soluble excipient.

12. A package comprising:
a solid composition comprising a reduced coenzyme Q10 Form II crystal, an emulsifier having an HLB of 6.0 or more, and an antioxidant;
a gas phase having a relative humidity of less than 50%; and
a container enclosing the solid composition and the gas phase.

13. The package according to claim 12, wherein the gas phase is air.

14. The package according to claim 12 or 13, wherein the emulsifier is one or more selected from the group consisting of: ester compounds of polyols and fatty acids optionally having substituents, wherein the polyols are selected from the group consisting of monoglycerol, polyglycerol, sorbitan, polyoxyethylene sorbitan, sucrose, propylene glycol, polypropylene glycol, ethylene glycol, and polyethylene glycol; and lecithin.

15. The package according to any one of claims 12 to 14, wherein the antioxidant is one or more selected from the group consisting of ascorbic acid, ascorbic acid salts, erythorbic acid, and erythorbic acid salts.

16. The package according to any one of claims 12 to 15, wherein the solid composition comprises 1 part by weight or more and 9900 parts by weight or less of the emulsifier with respect to 100 parts by weight of the crystal.

17. The package according to any one of claims 12 to 16, wherein the amount of the emulsifier contained in the solid composition is 1% by weight or more and 99% by weight or less.

18. The package according to any one of claims 12 to 17, wherein the solid composition further comprises a binder.

19. The package according to claim 18, wherein the binder is one or more selected from the group consisting of hydroxypropyl cellulose and hydroxypropylmethyl cellulose.

20. The package according to claim 18 or 19, wherein the solid composition is a particulate solid composition.

21. The package according to any one of claims 12 to 20, wherein the solid composition is free of a carbonate containing a sodium cation and/or a calcium cation.

22. The package according to any one of claims 12 to 21, wherein the solid composition is not a composition in which an oily component containing reduced coenzyme Q10 and a lipophilic antioxidant is polydispersed in domain form in a matrix containing a water-soluble excipient.
